# EUROPÄISCHE PATENTSCHRIFT

(11) **EP 4 753 596 B1**
(45) Veröffentlichungstag und Bekanntmachung des Hinweises auf die Patenterteilung: **18.11.2026**
(21) Anmeldenummer: 25780158.9
(22) Anmeldetag: 29.09.2025
(51) Int. Cl.: A61B 17/70, A61B 17/80

(54) **ANTERIORE ZERVIKALE PLATTE**
ANTERIOR CERVICAL PLATE
PLAQUE CERVICALE ANTÉRIEURE

(30) Priorität: 01.10.2024 DE 102024128449
(43) Veröffentlichungstag der Anmeldung: 10.06.2026
(73) Patentinhaber: AESCULAP AG, 78532 Tuttlingen (DE)
(72) Erfinder: STERN, Andreas, 78087 Mönchweiler (DE); SCHULTZ, Robert, 78532 Tuttlingen (DE); DIETRICH, Nils, 78187 Geisingen (DE); MIHARA, Hisanori, Yokohama-shi, Kanagawa 227-0044 (JP)
(74) Vertreter: Winter, Brandl - Partnerschaft mbB
(86) Internationale Anmeldenummer: PCT/EP2025/077777
(87) Internationale Veröffentlichungsnummer: WO 2026/073850

(56) Entgegenhaltungen:
- US-A1- 2006 064 097
- US-A1- 2009 306 667
- US-A1- 2019 314 068
- US-A1- 2021 290 276

## Beschreibung

### Technisches Gebiet

Die vorliegende Offenbarung betrifft eine anteriore zervikale Platte mit zumindest einem Verriegelungselement für eine darin eingesetzte Knochenschraube. Solche Systeme dienen zur mono- und multisegmentalen Stabilisierung der Halswirbelsäule.

Es sind verschiedene zervikale Plattensysteme auf dem Markt, die sich unter anderem hinsichtlich ihrer Verriegelungsmechanismen für die eingesetzten Knochenschrauben unterscheiden. Solche Plattensysteme werden bereits von der Anmelderin unter dem Namen Quintex und beispielsweise von den Firmen Bricon (unter dem Namen Shark CP Pro), Zimmer (Trinica), Medtronic (Zevo), Stryker (Ozark), Biomet (Maxan), DePuy Synthes (Skyline), GlobusMedical (Assure), Nuvasive (C360) und Biomed (Quantum) vertrieben.

Bei dem zervikalen Plattensystem gemäß US 8,906,077 B2 bzw. EP 2 217 163 B1 kommt ein Sperrelement in Verbindung mit einem gesonderten Fixierelement zum Einsatz.

Bei dem System gemäß US 7909,859 B2 werden die Schrauben mittels Klammern vor dem Herauswandern gesichert.

Bei dem System nach US 8,500,737 B2 wird entweder ein in einer Längsführung verschiebbarer Schnappring oder eine verschiebbare Platte für die Sicherung der eingesetzten Knochenschraube verwendet.

Die aus dem Dokument US 10,492,836 B2 bekannte zervikale Platte ist mit einer Verriegelung in der Ausgestaltung einer Art Flügelschraube, die mittels einer von der Rückseite der zervikalen Platte eingesetzten Spannmutter an der zervikalen Platte fixiert ist. Ein ähnliches Verriegelungselement ist beim System gemäß US8,480,717 B2 verwendet.

Ein Schraubensicherungssystem gemäß DE 202 21 560 U1 verwendet eine auf der Unterseite des betreffenden Schraubenkopfs verschiebbare Scheibe mit Langloch.

Das Verriegelungssystem gemäß US 8,778,001 B2 sieht Verriegelungsplatten vor, die mittels federnder Spreizbeine in einer hinterschnittenen Nut der zervikalen Platte gefangen sind.

Das Plattensystem gemäß US 8,747,441 B2 verwendet als Verriegelungselemente federnde C-Ringe oder geschlitzte Ringe, die in einer entsprechenden Nut der zervikalen Platte aufgenommen sind.

Die Verriegelung gemäß US 8,702,766 B2 verwendet pro Schraube einen verdrehbaren Plattenkörper mit einem radial und in Umfangsrichtung auskragenden Federarm, an dessen distalem Ende ein Klauenkörper sitzt. Der Klauenkörper wird zur Verriegelung beim Verdrehen des Plattenkörpers unter Biegeverformung des Federarms in eine hinterschnittene Führungsnut in der Platte gezwängt und schnappt bei Erreichen der Verriegelungsposition in eine Ausnehmung der Platte. Dieses Konzept verlangt aufgrund der Hinterschneidung eine größere Plattendicke.

Das zervikale Plattensystem gemäß US11,166,755 B2 sichert zwei benachbarte Knochenschrauben mittels über den betreffenden Schraubenkopf schnappbarer Federringe, die mit einer seitlich zwischen benachbarten Schraubenlöchern gelagerten Stellschraube aufweitbar sind.

Bei dem aus dem Dokument EP 1 737 365 B1 bekannten, gattungsbildenden zervikalen Plattensystem ist das Verriegelungselement für die Schrauben von einer federnden Platte gebildet, die in Längsführung aufgenommen ist.

Herkömmliche zervikale Platten, die regelmäßig mit einem Verriegelungssystem ausgestattet sind, müssen mit einer Dicke ausgeführt werden, die eine sichere und wenig störende Aufnahme der Verriegelungskomponenten erlaubt. Die Dicke der zervikalen Platte kann auch dadurch nicht beliebig verringert werden, u.a. deshalb, weil die Platte beim Einsetzen durch an der Platte ansetzender Handhabungsintrumente sicher gefasst werden muss. Es zeigt sich, dass solche bekannten zervikalen Platten, die in der Regel von randseitig leicht verrundeten Rechteckplatten gebildet sind, dann, wenn sie eingesetzt sind, zu Schluckbeschwerden führen.

Derartige, randseitig verrundete zervikale Platten sind aus den Dokumenten US 2019/314068 A1, US 2006/064097 A1 und US 2009/306667 A1 bekannt. Die Verrundung ist in einem Bereich eines die Platte in seitlicher Richtung verstärkenden Auges ausgebildet, in dem eine Knochenschraube aufzunehmen ist, was zu einer Erhöhung der Steifigkeit der Platte führt.

Gemäß US 2019/314068 A1 beginnt die Verrundung etwa im Abstand der Plattendicke von einer Randkante.

Bei der Platte gemäß US 2009/306667 A1 ist eine randseitige schräge Abkantung vorgesehen.

Gemäß US 2006/064097 A1 liegt eine randseitige Plattenverjüngung vor, die etwa in einem randseitigen Abstand beginnt, der größer ist als die Plattendicke. Allerdings verjüngt sich die Platte nicht hin zu einem randseitigen Rundungsabschnitt. Vielmehr ist die Kontur der Platten gemäß US 2006/064097 A1 und US 2009/306667 A1 so gestaltet, dass sie zwischen den randseitigen Augen zur Aufnahme der Knochenschrauben eine konkave Einbuchtung hat.

Der Anmeldung liegt die Aufgabe zugrunde, eine anteriore zervikale Platte mit Verriegelungsoption für eingesetzte Knochenschrauben zu schaffen, mit der unter Aufrechterhaltung einer ausreichenden Stabilität bei guter Handhabungsmöglichkeit beim Einsetzen der Platte Schluckbeschwerden besser vermieden werden können.
Diese Aufgabe wird mit einer anterioren zervikalen Platte gemäß Patentanspruch 1 gelöst. Demnach wird die zervikalen Platte mit einer im Wesentlichen gleichbleibenden Normaldicke **DN,** d.h. der Rohdicke der Platte ohne Bearbeitungsflächen, zumindest an ihrem kranialen Endabschnitt beginnend in einem Abstand AK von einer kranialen Randkante, für den gilt: $1 \times DN \leq AK \leq 2 , 5 \times DN$ auf der Basis eines kartesischen Koordinatensystems mit einer x-Achse in Längsrichtung, einer Y-Achse in Querrichtung und einer Z-Achse in Dickenrichtung der Platte in Richtung ihrer Dickenerstreckung bis zu einem in X-Richtung randseitigen Rundungsabschnitt auf 25% bis 80% der Normaldicke DN verjüngt, wobei die Normaldicke der Platte in einem Bereich zwischen 1 und 3 mm liegen kann. Dieser Verjüngung ist eine weitere Verjüngung der anterioren zervikalen Platte in Querrichtung überlagert, die von einer Verrundung ausgehend von einem Mittenbereich der randseitigen Aufnahmedurchbrüche bis zum Plattenende, d.h. bis zur Randkante der Platte hin gebildet ist, wobei sich das Ende der Platte ergibt, indem die beiden seitlichen Radien R der Verrundungen durch einen dritten, größeren Radius verbunden werden, so dass sich eine leicht konvex gekrümmte Randkante ergibt, deren Krümmungsradius ein Vielfaches des Radius der Verrundung beträgt..

Neuerungsgemäß bekommt also die zervikale Platte zumindest im kranialen Endbereich eine neue Formgebung, nämlich an den Enden der Platte eine geometrisch abgeflachte und verrundete Form, wodurch dafür gesorgt wird, dass Gewebe möglichst widerstandsfrei darüber gleiten kann. Somit kann post-operativen Schluckbeschwerden wirksam vorgebeugt werden, ohne dabei die stabilisierende Funktion für die Wirbelkörper außerhalb des Endbereichs zu schwächen

Es ist von zusätzlichem Vorteil, wenn die anteriore zervikale Platte in einem Zentralbereich eine hinterschnittene Instrumentenschnittstelle ausbildet. Denn diese Ausgestaltung hat zur Folge, dass ein chirurgisches Einsetzinstrument für die Platte in diese Instrumentenschnittstelle eingesetzt werden kann, ohne dass es über eine Randkante der Platte vorsteht. Es kann dadurch wirksam verhindert werden, dass das chirurgische Einsetzinstrument mit Knochensubstanz oder anderen Bereichen der Wunde kollidiert. Gleichzeitig ergibt sich der zusätzliche Vorteil, dass diese Instrumentenschnittstelle dazu genutzt werden kann, um einen Pin für die temporäre Fixierung der zervikalen Platte an den Wirbelkörpern aufzunehmen, damit die zervikalen Platte einfacher am Patienten ausgerichtet werden kann.

Die neue Gestaltung der zervikalen Platte kann für den Fall, dass sie mit Funktionsflächen für die Aufnahme einer Verriegelungsvorrichtung für gesetzte Schrauben ausgestattet werden soll, zusätzlich unterstützt werden, wenn die Funktionsflächen auf zumindest eine Bohrung zur Aufnahme eines Lagerzapfens zumindest einer mit Handkraft verschwenkbaren Verriegelungsscheibe beschränkt sind.

Die Stabilität der zervikalen Platte wird in Bereichen außerhalb ihrer Endabschnitte dann besonders groß, wenn der Durchmesser einer Bohrung zur Aufnahme des Lagerzapfens für die Drehachse eines Verriegelungskörpers auf Werte im Bereich zwischen 1,2 und 3,5 mm beschränkt bleibt.

Besonders einfach kann die hinterschnittene Instrumentenschnittstelle dann ausgebildet und hergestellt werden, wenn sie schlüssellochartig ausgeformt und zu einem ohnehin regelmäßig in der zervikalen Platte ausgebildeten Fenster offen ist, wodurch der Eingriff des chirurgischen Einsetzinstruments zusätzlich erleichtert wird.

Wenn sich die zervikale Platte im kranialen Endabschnitt in Richtung der Z-Achse, also in Dickenrichtung zunächst leicht bogenförmig und beginnend mit einer Kante, die im Wesentlichen mittig im Endabschnitt liegt, linear zum Rundungsabschnitt verjüngt, kann über die Lage der Kante die gewebeschonende Funktion bestmöglich angepasst werden.

Nachstehend werden anhand schematischer Zeichnungen Ausführungsbeispiele der neuen anteriore zervikalen Platte näher beschrieben. Es zeigen:

### Kurzbeschreibung der Figuren

Fig. 1 eine perspektivische Ansicht einer zervikalen Platte von unten;
Fig. 2 eine perspektivische Ansicht der zervikalen Platte der Figur 1 von oben;
Fig. 3 die vergrößerte perspektivische Ansicht eines Endabschnitts der zervikalen Platte gemäß Fig. 1 und 2;
Fig. 4 eine Längs-Schnittansicht einer hinsichtlich der in ihr ausgebildeten Durchbrüche etwas modifizierten zervikalen Platte im Bereich ihres kranialen Endabschnitts;
Fig. 5 eine teilweise aufgebrochene Seitenansicht der zervikalen Platte gemäß Figur 4;
Fig. 6 die Draufsicht einer hinsichtlich ihrer Segmente modifizierten zervikalen Platte;
Fig. 7 die Seitenansicht der zervikalen Platte gemäß Figur 6; und
Fig. 8 die Ansicht der zervikalen Platte gemäß Fig. 6 von unten

### Beschreibung der Ausführungsbeispiele

Nachstehend werden Ausführungsbeispiele der vorliegenden Offenbarung auf der Basis der zugehörigen Figuren beschrieben.

### Erstes Ausführungsbeispiel

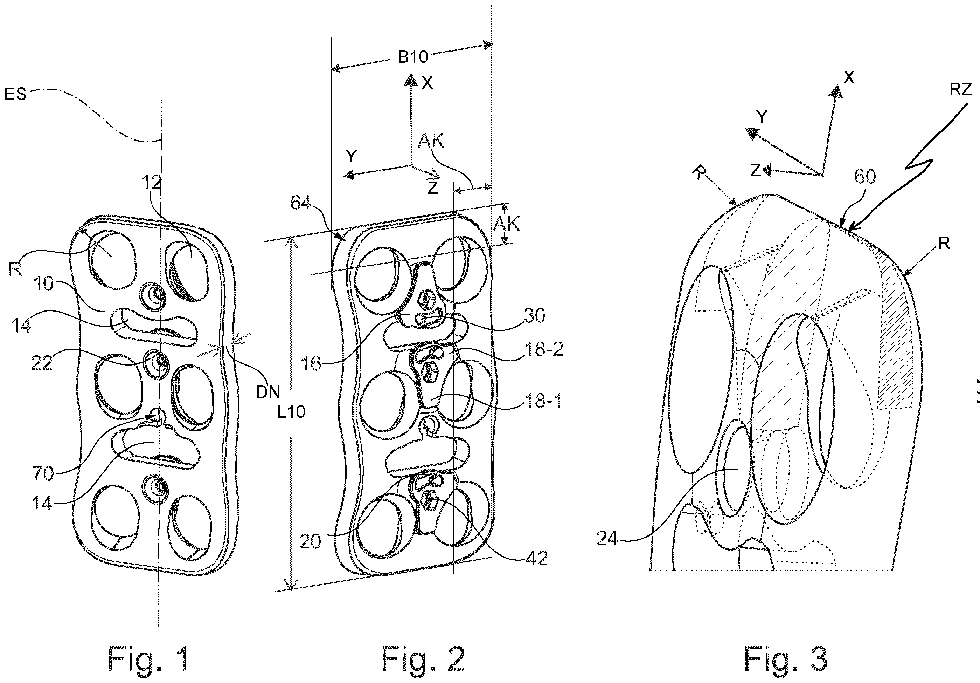

In den Figuren 1 bis 3 ist ein erstes Ausführungsbeispiel einer anterioren zervikalen Platte 10 gezeigt. Die zervikale Platte 10 ist symmetrisch zu einer in Figur 1 schematisch eingezeichneten Symmetrieebene ES und hat 6 annähernd kreisrunde Aufnahmedurchbrüche 12 für nicht näher dargestellte Knochenschrauben und pro Segment jeweils ein Fenster 14. Zur Beschreibung der Geometrie der zervikalen Platte 10 wir ein in den Figuren jeweils eingetragenes kartesisches Koordinatensystem mit den Achsen X, Y und Z herangezogen, bei dem sich die X-Achse in der Längsrichtung, die Y-Achse in Querrichtung und die Z-Achse in Dickenrichtung der zervikalen Platte 10 erstreckt.

Anlageflächen für die Köpfe der Knochenschrauben sind nicht näher beschrieben, sie sind vorzugsweise so ausgebildet, dass sie aus einer Kugelfläche oder einer Kugelfäche in Kombination mit einer Kegelfläche bestehen, wodurch sowohl winkelmäßig eingeschränkte (constrained) Schrauben, als auch winkelmäßig variable Schrauben verwendet werden können. Die gezeigte zervikale Platte 10 stellt damit eine sogenannte Zwei-Segment-Hybridplatte dar, bei der ein sogenanntes translatorisches Schraubengleiten verhindert ist.

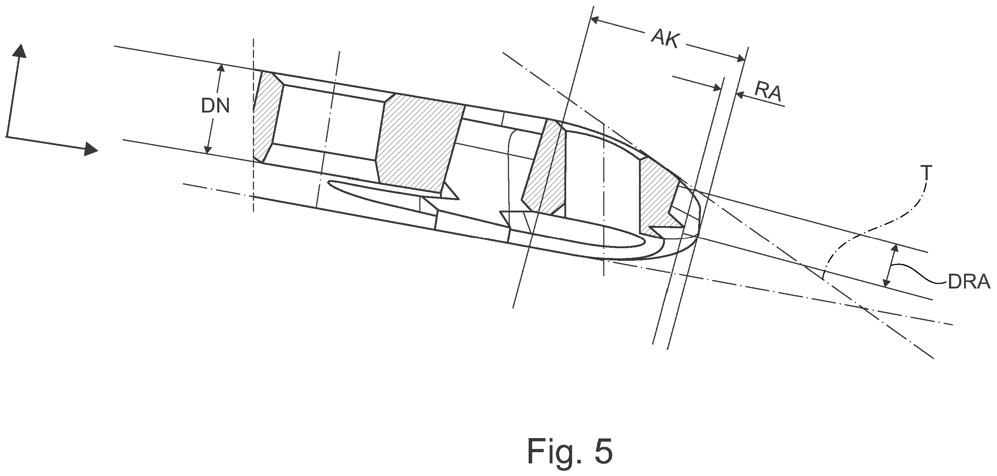

In an sich bekannter Weise ist die zervikale Platte 10 mit einer in Z-Richtung gemessenen Normaldicke DN, also einer Dicke, die am Rohling ohne irgendwelche Bearbeitungsflächen gemessen wird, in Längsrichtung vorgebogen (wie aus Fig. 1 und 2 hervorgeht) und - wie aus der Schnittansicht gemäß Figur 5 erkennbar - in Querrichtung leicht gewölbt, um sich möglichst eng an die Form der Halswirbelsäule anzuschmiegen.

Die zervikale Platte 10 ist mit einem Verriegelungsmechanismus für die gesetzten und mit den Halswirbeln verschraubten Knochenschrauben ausgestattet. Durch diesen Verriegelungsmechanismus werden die Knochenschrauben an einer Migration der Schrauben aus der Platte heraus gehindert.

Der Verriegelungsmechanismus ist so ausgebildet, dass ein Verriegelungskörper 16 aus einer in Figur 2 dargestellten einen Aufnahmedurchbruch 12 frei zugänglich lassenden Position in eine Verriegelungsposition bringbar ist, in der der im Aufnahmedurchbruch 12 liegende Schraubenkopf vom Verriegelungskörper 16 zumindest teilweise abgedeckt ist und so die Migration der Schraube verhindert wird.

Im gezeigten Ausführungsbeispiel sind drei Verriegelungskörper 16 vorgesehen, die jeweils zwei benachbarten Aufnahmedurchbrüchen 12 zugeordnet und jeweils im Wesentlichen mittig zwischen zwei benachbarten Aufnahmedurchbrüchen 12 angeordnet sind. Jeder Verriegelungskörper 16 ist von einer drehbar um eine im Wesentlichen senkrecht auf der zervikalen Platte 10 stehende Drehachse A16 an der anterioren zervikalen Platte 10 festgelegten dünnen Scheibe 16 gebildet, die über den Umfang winkelmäßig verteilt zumindest einen Verriegelungsarm 18-1 und 18-2 und einen sich um einen bestimmten Zentriwinkel erstreckenden Stützabschnitt 20 aufweist, der durch Verdrehen der Scheibe 16 um einen vorbestimmten Drehwinkel aus der in Figur 2 dargestellten geöffneten Position in eine die Knochenschrauben verriegelnde Rastposition schnappt.

Die Verdrehbarkeit der Scheibe 16 wird durch einen vorzugsweise einstückig mit der Scheibe 16 ausgebildeten hohlen Lagerzapfen 22 bereitgestellt, der mit Spielpassung in einer Bohrung 24 (siehe Figur 3) der zervikalen Platte 10 aufgenommen und darin auf der Unterseite der Platte 10 verstemmt ist. Der Verriegelungskörper in der Ausgestaltung als Scheibe 16 mit hohlem Lagerzapfen 22, der eine Mehrkantausnehmung 42, z.B. eine Innensechskantausnehmung für ein Betätigungswerkzeug, z.B. einen Schraubendreher hat, ist auf diese Weise kanülliert, was dazu genutzt werden kann, den Lagerzapfen 22 als Führung für einen temporären Fixationspin zum Fixieren der zervikalen Platte 10 an der Wirbelsäule heranzuziehen.

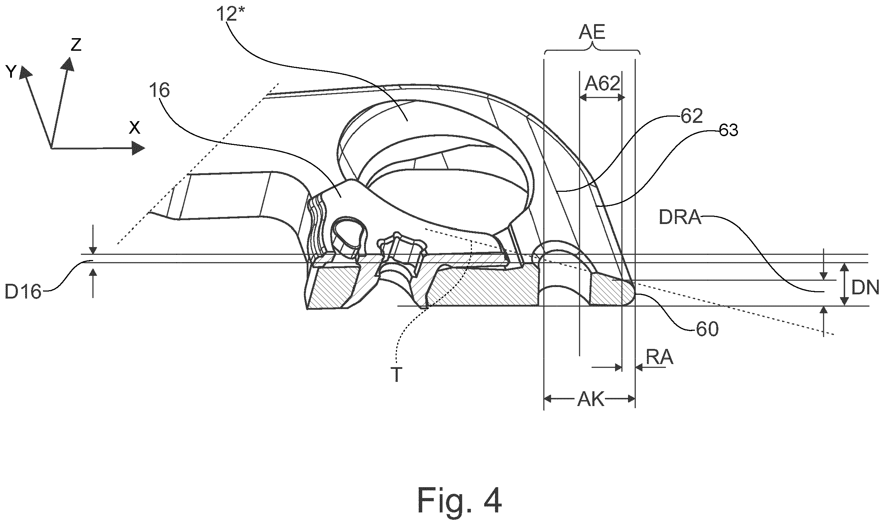

Die Abmessungen der Scheibe 16 sind so gewählt, dass sie die Gesamtdicke des zervikalen Plattensystem nur minimal anheben. Beispielsweise liegt die Dicke D16 (siehe Fig. 4) der Scheibe 16 für den Fall, dass die Normaldicke **DN** der zervikalen Platte 10 zwischen 1,5 und 2,5 mm beträgt, im Bereich zwischen 0,25 und 0,75 mm. Der Durchmesser D22 des Lagerzapfens 22 liegt in diesem Fall im Bereich zwischen 1,2 und 3,5 mm.

Eine Besonderheit des Verriegelungsmechanismus liegt darin, dass es einen Rastmechanismus integriert. Hierzu wirkt der Stützabschnitt 20 mit einem auf der zervikalen Platte 10 angebrachten Rastpin 30 zusammen, der beim Verdrehen der Scheibe 16 den Stützabschnitt 20 vor Erreichen der Rastposition verformt, bevor die Scheibe 16 in die Rastposition schnappt.

Eine weitere Besonderheit der anterioren zervikalen Platte besteht darin, dass sich die zervikale Platte 10 mit einer in Figur 4 bezeichneten Normaldicke **DN** zumindest an ihrem kranialen Endabschnitt AE in Richtung der Z-Achse, also in ihrer Dickenerstreckung in besonderer Weise verjüngt. Die Verjüngung der anterioren zervikalen Platte in Querrichtung erfolgt über eine Verrundung ausgehend von ungefähr der Mitte der Durchbrüche 12 bzw. Bohrungen bis zum Ende der Platte hin. Die Platte nimmt also an ihren Enden sowohl hinsichtlich ihrer Breite als auch hinsichtlich ihrer Dicke kontinuierlich ab. Diese Verjüngung beginnt für die Dicke in einem Abstand AK (siehe Fig. 2 und 4) von einer kranialen Randkante 60, wobei für den Abstand AK gilt: $1 \times DN \leq AK \leq 2 , 5 \times DN$

Dabei verjüngt sich die Dicke der zervikalen Platte 10 - wie am besten in der Figur 4 gezeigt - in Längsrichtung, also entlang der X-Richtung so, dass sie an einem randseitigen Rundungsabschnitt RA, der zwischen 10 und 30% des kranialen Endabschnitts ausmacht, nur noch 25% bis 80% der Normaldicke DN beträgt. Diese Dicke ist in Figur 4 mit DRA bezeichnet. Der Verjüngungsquerschnitt in einem Längsschnitt betrachtet, also in einem Schnitt durch eine zur X-Achse parallelen Mittelebene der Platte ist in Figur 3 grob schraffiert gekennzeichnet, die sich in Richtung der Y-Achse, also in Querrichtung erstreckende Verjüngungsoberfläche ist feiner schraffiert markiert.

In den Figuren 4 und 5 ist die Geometrie der Formgebung des kranialen Endabschnitts im Einzelnen dargestellt.

Die zervikale Platte, die mit einer kleinen Normaldicke im Bereich zwischen 1,5 und 2,5 mm ausgeführt ist, verjüngt sich beginnend im Abstand AE von der Randkante 60, also beim Ausführungsbeispiel gemäß Figur 4 etwa die im Bereich des kranialen Aufnahmedurchbruchs 12*, zunächst leicht bogenförmig bis zu einer ersten Kante 62, die im Wesentlichen mittig im Endabschnitt AE liegt. Der Abstand der ersten Kante 62 vom Randabschnitt RA ist in Figur 4 mit A62 bezeichnet und liegt im Bereich zwischen 0,1 und 0,55 x AK.

Von dort nimmt die Dicke der Platte in X-Richtung, also in Längsrichtung vorzugsweise linear ab, bis an einer zweiten geraden Kante 63 der Rundungsabschnitt RA mit einer Dicke DRA erreicht ist, die nur noch 25 bis 80% der Normaldicke DN (siehe Figur 4, in der die mit strichpunktierten Linien gezeigten Bezugslinien auf der unteren bzw. oberen Oberfläche der Platte liegen) der zervikalen Platte 10 ausmacht. Dieser lineare Verlauf bis zum Rundungsabschnitt RA ist in Figur 4 durch die Tangente T angedeutet.

Die Verjüngung des Endabschnitts der zervikalen Platte 10 in Richtung der Y-Achse, also in Querrichtung, d.h. die Abnahme der Breite der Platte, ist - wie am besten aus den Figuren 1 und 2 ersichtlich - von einer von einer Verrundung 64, beispielsweise mit einem Radius R gebildet ist. Die Verrundung 64 überlagert sich also mit der Verringerung der Dicke in Richtung der X-Achse, also in Längsrichtung. Das Ende der Platte ergibt sich, indem die beiden Radien R der Verrundungen 64 durch einen dritten, größeren Radius RZ (siehe Figur 3) verbunden werden, so dass sich eine leicht konvex gekrümmte Randkante 60 ergibt. Der Krümmungsradius RZ der Randkante 60 beträgt ein Vielfaches des Radius R der Verrundung 64. Wie dargestellt, nehmen also in Längsrichtung sowohl die Breite der Platte (ΔY) als auch die Dicke der Platte (ΔZ) ab. In Querrichtung, also in Richtung der Y-Achse, ist die Verrundung mit den Radien R und RZ bis zur Mittelebene EM der Platte vorgesehen, die sich mit der in Z-Richtung gerichteten Verjüngung entlang der X-Achse überlagert.

Die an der zervikalen Platte 10 erforderlichen Funktionsflächen für die Halterung der Verriegelungsvorrichtung für gesetzte Schrauben, also der Verriegelungskörper 16 in der Ausgestaltung der Scheiben mit einem Lagerzapfen 22, sind auf die Bohrung 24 zur Aufnahme des Lagerzapfens 22 beschränkt. Es hat sich gezeigt, dass der Durchmesser der Bohrung 24 zur Aufnahme des Lagerzapfens 22 im Bereich zwischen 1,2 und 3,5 mm liegen kann.

An der zervikalen Platte 10 sind zusätzlich Vorkehrungen für eine die Platte mechanisch gering beanspruchende Handhabung durch ein chirurgisches Einsetzinstrument getroffen. Hierzu bildet die Platte 10 in einem Zentralbereich eine hinterschnittene Instrumentenschnittstelle 70 aus, über die die zervikale Platte 10 an ein chirurgisches Einsetzinstrument gekoppelt werden kann. Dadurch ergibt sich der Vorteil, dass da Einsetzinstrument nicht mehr über den Rand der zervikalen Platte vorsteht, wodurch Kollisionen des Instruments mit Knochen vermieden werden. Gleichzeitig können Ausnehmungen der Instrumentenschnittstelle 70 für das Setzen von temporären Fixationspins herangezogen werden, mit denen eine einfache und exakte Ausrichtung der zervikalen Platte an der Halswirbelsäule unterstützt wird.

Wie in Figur 1 und 2 gezeigt, ist die hinterschnittene Instrumentenschnittstelle 70 schlüssellochartig ausgebildet ist und zu einem Fenster 14 der zervikalen Platte 10 offen.

### Zweites Ausführungsbeispiel

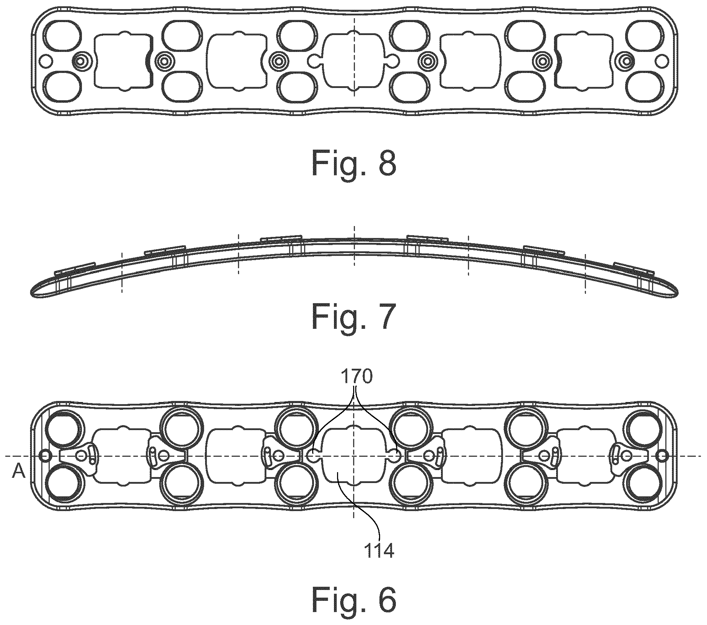

**In** den Figuren 6 bis 8 ist ein weiteres Ausführungsbeispiel der zervikalen Platte gezeigt, die das Bezugszeichen 110 hat. Die kranialen und dorsalen Endabschnitte sind ebenso wie die Verriegelungsvorrichtungen in gleicher Weise wie bei dem Ausführungsbeispiel der Figuren 1 bis 5 ausgeführt, so dass eine Beschreibung entfallen kann.

Die zervikale Platte 110 ist als hybride 5-Segment-Platte ausgeführt. Dementsprechend können an einem zentralen Fenster 114 zwei Instrumentenschnittstellen 170 ausgebildet sein.

Selbstverständlich sind Abwandlungen der beschriebenen Ausführungsbeispiele möglich, ohne den Grundgedanken der Neuerung zu verlassen.

Die Formgebung eignet sich gleichermaßen für anteriore zervikale Platten, die für constrained Versorgungen hergerichtet sind, und auch für sogenannte dynamische Platten für dynamische Versorgungen. Der Begriff "constrained" bedeutet dabei eine eingeschränkte Stabilität/ Steifigkeit der Verbindung zwischen Platte und Schraube, "dynamisch" bedeutet eine Beweglichkeit (rotatorisch und/ oder translatorisch) zwischen Schraube und Platte.

### Bezugszeichenliste

- 10: zervikale Platte
- 12: Aufnahmedurchbrüche
- 14, 114: Fenster
- 16: Verriegelungskörper als Scheibe
- D16: Dicke von 16
- 18-1, 18-2: Verriegelungsarme
- 20: Stützabschnitt
- 22: Lagerzapfen
- 24: Bohrung
- 30: Rastpin

- 42: Mehrkantausnehmung
- 60: Randkante
- 62: erste Kante
- A62: Abstand der Kante
- 63: zweite Kante
- 64: Verrundung
- 70, 170: Instrumentenschnittstelle
- DN: Normaldicke
- AE: Endabschnitt
- AK: Abstand
- RA: Rundungsabschnitt
- DRA: Dicke von RA
- T: Tangente
- ES: Symmetrieebene

## Patentansprüche

1. Anteriore zervikale Platte (10) mit Aufnahmedurchbrüchen (12) für Knochenschrauben und gegebenenfalls zwischen den Aufnahmedurchbrüchen (12) liegenden Fenstern (), **dadurch gekennzeichnet, dass** sich die zervikale Platte (10; 110) mit einer Normaldicke DN, d.h. der Rohdicke der Platte ohne Bearbeitungsflächen, zumindest an ihrem kranialen Endabschnitt (AE) beginnend in einem Abstand AK von einer kranialen Randkante (60), für den gilt: $1 \times DN \leq AK \leq 2 , 5 \times DN$ auf der Basis eines kartesischen Koordinatensystems mit einer X-Achse in Längsrichtung, einer Y-Achse in Querrichtung und einer Z-Achse in Dickenrichtung der Platte (10) in Richtung ihrer Dickenerstreckung bis zu einem randseitigen Rundungsabschnitt (RA) auf 25% bis 80% der Normaldicke DN verjüngt, wobei dieser Verjüngung eine weitere Verjüngung der anterioren zervikalen Platte in Querrichtung überlagert ist, die von einer Verrundung (64) ausgehend von einem Mittenbereich der randseitigen Aufnahmedurchbrüche bis zur Randkante der Platte hin gebildet ist, und sich das Ende der Platte ergibt, indem die beiden seitlichen Radien R der Verrundungen (64) durch einen dritten, größeren Radius RZ verbunden werden, so dass sich eine leicht konvex gekrümmte Randkante (60) ergibt, wobei der Krümmungsradius (RZ) der Randkante (60) ein Vielfaches des Radius (R) der Verrundung (64) beträgt.

2. Anteriore zervikale Platte nach Anspruch 1, **dadurch gekennzeichnet, dass** die Platte (10; 110) in einem Zentralbereich zumindest eine hinterschnittene Instrumentenschnittstelle (70; 170) ausbildet.

3. Anteriore zervikale Platte nach Anspruch 1 oder 2, mit eingearbeiteten Funktionsflächen für die Aufnahme einer Verriegelungsvorrichtung für gesetzte Schrauben, **dadurch gekennzeichnet, dass** die Funktionsflächen auf zumindest eine Bohrung (24) zur Aufnahme eines Lagerzapfens (22) zumindest einer mit Handkraft verschwenkbaren Verriegelungsscheibe (16) beschränkt sind.

4. Anteriore zervikale Platte nach Anspruch 1, **dadurch gekennzeichnet, dass** die Normaldicke DN der Platte (10; 110) im Bereich zwischen 1,5 und 2,5 mm liegt.

5. Anteriore zervikale Platte nach Anspruch 3 oder 4, **dadurch gekennzeichnet, dass** der Durchmesser der Bohrung (24) zur Aufnahme des Lagerzapfens (22) im Bereich zwischen 1,2 und 3,5 mm liegt.

6. Anteriore zervikale Platte nach einem der Ansprüche 2 bis 5, **dadurch gekennzeichnet, dass** die hinterschnittene Instrumentenschnittstelle (70; 170) schlüssellochartig ausgebildet ist und zu einem Fenster (14; 114) der zervikalen Platte (10; 110) offen ist.

7. Anteriore zervikale Platte nach einem der Ansprüche 1 bis 6, **dadurch gekennzeichnet, dass** die Verjüngung in Querrichtung von einer Verrundung (64) mit einem Radius R gebildet ist.

8. Anteriore zervikale Platte nach einem der Ansprüche 1 bis 6, **dadurch gekennzeichnet, dass** die Verjüngung in Querrichtung von einer Verrundung (64) aus zumindest zwei Radien (R und RZ) gebildet ist.

9. Anteriore zervikale Platte nach einem der Ansprüche 1 bis 7, **dadurch gekennzeichnet, dass** sich die zervikale Platte (10) im kranialen Endabschnitt in Dickenrichtung zunächst leicht bogenförmig und beginnend mit einer Kante (62), die im Wesentlichen mittig im Endabschnitt (AE) liegt, linear zum Rundungsabschnitt (RA) verjüngt.

10. Anteriore zervikale Platte nach einem der Ansprüche 1 bis 8, **gekennzeichnet durch** die Ausgestaltung als hybride Platte für constrained Versorgungen oder als dynamische Platte für dynamische Versorgungen.

## Claims

1. An anterior cervical plate (10) with receiving openings (12) for bone screws and, where applicable, windows () situated between the receiving openings (12), **characterized in that** the cervical plate (10; 110), having a standard thickness DN, i.e., the raw thickness of the plate without working surfaces, tapers, at least at its cranial end section (AE), in the direction of its thickness extension up to an edge-side rounded section (RA) to 25 per cent to 80 per cent of the standard thickness DN, starting at a distance AK from a cranial edge (60), for which the following applies: $1 \times DN \leq AK \leq 2 , 5 \times DN$ on the basis of a Cartesian coordinate system with an X-axis in the longitudinal direction, a Y-axis in the transverse direction and a Z-axis in the thickness direction of the plate (10), the tapering being superimposed by a further tapering of the anterior cervical plate in the transverse direction which is formed by a round (64) extending from a central region of the edge-side receiving openings to the edge of the plate, and the end of the plate is formed by connecting the two lateral radii R of the rounds (64) by a third, larger radius RZ, so that a slightly convexly curved edge (60) results, wherein the radius of curvature (RZ) of the edge (60) is a multiple of the radius (R) of the round (64).

2. The anterior cervical plate according to claim 1, **characterized in that** the plate (10; 110) forms at least one undercut instrument interface (70; 170) in a central region.

3. The anterior cervical plate according to claim 1 or 2, comprising integrated functional surfaces for receiving a locking device for set screws, **characterized in that** the functional surfaces are limited to at least one bore (24) for receiving a bearing pin (22) of at least one locking disc (16) that can be pivoted by hand.

4. The anterior cervical plate according to claim 1, **characterized in that** the standard thickness DN of the plate (10; 110) lies in the range between 1.5 and 2.5 mm.

5. The anterior cervical plate according to claim 3 or 4, **characterized in that** the diameter of the bore (24) for receiving the bearing pin (22) is in the range of 1.2 to 3.5 mm.

6. The anterior cervical plate according to any of claims 2 to 5, **characterized in that** the undercut instrument interface (70; 170) is keyhole-shaped and opens toward a window (14; 114) in the cervical plate (10; 110).

7. The anterior cervical plate according to any of claims 1 to 6, **characterized in that** the taper in the transverse direction is formed by a round (64) with a radius R.

8. The anterior cervical plate according to any of claims 1 to 6, **characterized in that** the taper in the transverse direction is formed by a round (64) comprising at least two radii (R and RZ).

9. The anterior cervical plate according to any of claims 1 to 7, **characterized in that**, at its cranial end section, the cervical plate (10) tapers, initially slightly arched in the thickness direction and, starting from an edge (62) which lies substantially centrally within the end section (AE), linearly towards the rounded section (RA).

10. The anterior cervical plate according to any of claims 1 to 8, **characterized by** being designed as a hybrid plate for constrained fixations or as a dynamic plate for dynamic fixations.

## Revendications

1. Plaque cervicale antérieure (10) avec des ouvertures de réception (12) pour des vis à os et des fenêtres () se trouvant éventuellement entre les ouvertures de réception (12), **caractérisée en ce que** la plaque cervicale (10 ; 110) présente une épaisseur normale DN, c'est-à-dire l'épaisseur brute de la plaque sans surface d'usinage, au moins au niveau de sa section d'extrémité (AE) crânienne en commençant à une distance AK par rapport à un bord périphérique (60) crânien, pour laquelle s'applique ce qui suit : $1 \times DN \leq AK \leq 2 , 5 \times DN$ sur la base d'un système de coordonnées cartésien avec un axe X dans le sens longitudinal, un axe Y dans le sens transversal et un axe Z dans le sens de l'épaisseur de la plaque (10), celle-ci se rétrécit en direction de son étendue d'épaisseur jusqu'à une section d'arrondi (RA) côté bord sur 25 % à 80 % de l'épaisseur normale DN, dans laquelle à ce rétrécissement est superposé un autre rétrécissement de la plaque cervicale antérieure dans le sens transversal qui est formé par un arrondi (64) à partir d'une zone médiane des ouvertures de réception côté bord jusqu'au bord périphérique de la plaque, et il en résulte l'extrémité de la plaque en reliant les deux rayons latéraux R des arrondis (64) par un troisième rayon RZ plus grand de sorte qu'il en résulte un bord périphérique (60) courbé de manière légèrement convexe, dans laquelle le rayon de courbure (RZ) du bord périphérique (60) s'élève à un multiple du rayon (R) de l'arrondi (64).

2. Plaque cervicale antérieure selon la revendication 1, **caractérisée en ce que** la plaque (10 ; 110) forme au moins une interface d'instrument (70 ; 170) contre-dépouillée dans une zone centrale.

3. Plaque cervicale antérieure selon la revendication 1 ou 2, avec des surfaces fonctionnelles intégrées pour la réception d'un dispositif de verrouillage pour des vis placées, **caractérisée en ce que** les surfaces fonctionnelles sont limitées à au moins un alésage (24) pour la réception d'un tourillon (22) au moins d'un disque de verrouillage (16) pivotant à la force de la main.

4. Plaque cervicale antérieure selon la revendication 1, **caractérisée en ce que** l'épaisseur normale DN de la plaque (10 ; 110) se situe dans la plage comprise entre 1,5 et 2,5 mm.

5. Plaque cervicale antérieure selon la revendication 3 ou 4, **caractérisée en ce que** le diamètre de l'alésage (24) pour la réception du tourillon (22) se situe dans la plage comprise entre 1,2 et 3,5 mm.

6. Plaque cervicale antérieure selon l'une quelconque des revendications 2 à 5, **caractérisée en ce que** l'interface d'instrument (70 ; 170) contre-dépouillée est conçue à la manière d'un trou de serrure et est ouverte vers une fenêtre (14 ; 114) de la plaque cervicale (10 ; 110).

7. Plaque cervicale antérieure selon l'une quelconque des revendications 1 à 6, **caractérisée en ce que** le rétrécissement est formé dans le sens transversal par un arrondi (64) avec un rayon R.

8. Plaque cervicale antérieure selon l'une quelconque des revendications 1 à 6, **caractérisée en ce que** le rétrécissement est formé dans le sens transversal par un arrondi (64) d'au moins deux rayons (R et RZ).

9. Plaque cervicale antérieure selon l'une quelconque des revendications 1 à 7, **caractérisée en ce que** la plaque cervicale (10) se rétrécit tout d'abord légèrement en arc dans la section d'extrémité crânienne dans le sens de l'épaisseur et en commençant par une arête (62) qui se trouve sensiblement au milieu dans la section d'extrémité (AE), linéairement par rapport à la section d'arrondi (RA).

10. Plaque cervicale antérieure selon l'une quelconque des revendications 1 à 8, **caractérisée par** la configuration comme plaque hybride pour des alimentations contraintes ou comme plaque dynamique pour des alimentations dynamiques.
